# EUROPEAN PATENT APPLICATION

(11) **EP 1 666 048 A2**
(43) Date of publication of application: **07.06.2006**
(21) Application number: 04788526.4
(22) Date of filing: 17.09.2004
(51) Int. Cl.: A61K 35/39, A61K 38/28, A61P 3/10, C07K 14/625

(54) **METHOD FOR PRODUCING INSULIN FROM A NATURAL SOURCE AND INSULIN**

(30) Priority: 18.09.2003 RU 2003127995
(71) Applicant: Tsygankov, Vladimir Vladimirovich, Kaluzhskaya obl., 577298 (RU)
(72) Inventor: Tsygankov, Vladimir Vladimirovich, Kaluzhskaya obl., 577298 (RU)
(74) Representative: Dunne, Emma Louise
(86) International application number: PCT/RU2004/000365
(87) International publication number: WO 2005/026315

(57) **Abstract**

The inventive method for producing insulin consists in using a reindeer pancreas which is homogenized in an ethanol hydrochloride solution, whereupon extraction is effected followed by clarifying the solution to obtain a supernatant fluid which is subjected to an ion-exchange chromatography and isoelectric precipitation to produce insulin as the desired product which is purified by a high-efficiency reversed-phase liquid co-chromatography. The resultant insulin is capable of competing for a bond with an insulin receptor at a concentration over 100 ng/ml.

## Description

### Technical Field

The present invention relates to medicine, particularly to producing insulin preparations applicable for therapy of diabetes mellitus.

### Background Art

At present use is made for treating diabetes mellitus of a broad spectrum of insulin preparations produced from natural sources, i.e., pancreatic islands of large horned cattle and swine. Both bovine and porcine insulins that are closest to human insulin as to their structure and amino acid sequence, display such an activity in human organism that is comparable with activity of human insulin. Bovine insulin differs from human one in three positions in the amino acid sequence, whereby it is featured by rather high immunogenicity and therefore is but in rare use nowadays. On the other hand, a molecule of porcine insulin which is derived by extraction from the pancreas of swine (cf. Greater Medical Encyclopaedia, the entry "Insulin") differs from human insulin only in a single amino acid in the B-chain (that is, having alanine at position 30 instead of threonine), thereby being in rather widespread use due to modern purification techniques. However, upon a prolonged use antibodies to porcine insulin start accumulating in human organism, thereby bringing to naught its therapeutic effect thereon. Furthermore, the fastest-acting insulin produces its maximum effect in a rather long lapse of time, that is, from 2 to 8 hours.

Nevertheless, it is due to an ever-growing incidence rate of diabetes mellitus that a material substrate of the porcine insulin (i.e., the pancreas of swine) is liable to get in deficit before long. That is why, despite an undoubtful high quality of multicomponent porcine insulins applicable to good advantage nowadays, in the long view one must seek for changing over to alternative sources of insulin production.

### Summary of the Invention

The desired technical result of the present invention resides in extending the range of natural sources of production of insulin able to compete, in a dosage-dependent matter, for a bond with an insulin receptor, to cause, when in a concentration exceeding 100 ng/ml, an abrupt increase in receptor bonding and featuring higher hydrophobicity than standard insulins have and hence some definite differences in the structure of a molecule thereof.

The aforesaid technical result is achieved due to the fact that in a method for producing insulin by its being isolated from the pancreas of a natural source, according to the invention, use is made of a reindeer pancreas for the purpose, which pancreas is homogenized in an ethanol hydrochloride solution and extracted, whereupon the solution is clarified to obtain a supernatant fluid which is then subjected to ion-exchange chromatography and isoelectric precipitation to produce desired insulin. Finally, the thus-produced insulin is purified by being subjected to high-efficiency reversed-phase liquid co-chromatography.

### Brief Description of the Drawings

In what follows the present invention is exemplified by the disclosure of a specific embodiment thereof to be had in conjunction with the accompanying drawings, wherein according to the invention:
FIG. 1 illustrates a reversed-phase liquid co-chromatography of a mixture of standard bovine insulin, porcine insulin, and reindeer pancreas-derived insulin;
FIG.2 illustrates characteristic curves representing competitive displacement of reindeer pancreas-derived insulin radiodinated with ¹²⁵I and bonded with a homologous receptor of the rat liver plasma membranes.

### Description of the Preferred Embodiment

The herein-proposed method for producing insulin is carried into effect as follows.

A reindeer pancreas comprising alongside with endocrine tissue also a great amount of fatty interlayers, is homogenized in an ethanol hydrochloride solution (1:15). Extraction is carried out in a shaker for a two-hour period at room temperature, whereupon the solution is centrifugation-clarified for 60 min at 3000 rpm and 4°C to obtain a supernatant fluid.

### Ion-exchange chromatography

The supernatant fluid of the pancreas is subjected to ion-exchange chromatography. To this aim, the supernatant fluid is applied to a 5x50 cm column packed with KY-23 microporous sulfocationite in the H⁺ form. Further purification steps consist in defatting cationic protein with 70-percent ethanol, washing-off ballast proteins with 0.5 M ammonium acetate, pH=5.0, and elution of insulin with 0.2 N ammonium buffer, pH=9.4. The composition of eluates is monitored in a spectrophotometer at a wavelength of 280 nm. The protein peak identified under the same conditions as in the standard insulin is gathered and, after having been reduced to pH=2.3, undergoes rechromatography.

### Isoelectric precipitation

The proteins concomitant with the principal matter are made to precipitate by successive reduction of the pH value of the eluate resultant from ion-exchange chromatography to 2.0, and further upon adding acetone (20% of a total amount of the solution), to 4.0. Next the solution is centrifugalized, and the centrifugate is frozen. Thereupon a 10-percent zinc- acetate solution (2% of a total amount of the solution) is added to the supernatant fluid to bring the pH value of the solution to 5.95, and the solution is let to stand at +20°C for an hour, whereupon is put in a cooler for 72 hours. The precipitate is centrifugation-separated in model K-23 at 4000 rpm or in model K-70 at 3000 rpm for 60 min at 4°C.

### High-efficiency reversed-phase liquid co-chromatography

Insulin produced from reindeer pancreas is purified by high-efficiency reversed-phase liquid co-chromatography using the Knauer System (Germany) in a Diaspher C column measuring 18.5 µm x 4.0 x 250 mm (available from "ELSIKO", Russia) using buffer mixtures, at an elution rate of 1 ml/min. An optical density of the eluate is recorded at a 226 nm wavelength. Significant peaks are subjected to rechromatography under conditions similar to those of fractionation.

### Radioligand assays

Insulin isolated from reindeer pancreas is tested for ability to get bonded with an insulin receptor in a radioreceptor system (Rusakov et al., "Isolation and characterization of insulin in Russian sturgeon *(Acipenser guldenstaedti"*, J. Peptide Res., 1988, v.51, pp.395-400) specific for the hormone involved. The system comprises radiolabeled insulin, plasma membranes of the liver and standard non-labeled hormone. An assay consists in constructing the curves representing competitive displacement of ¹²⁵I-insulin bonded with plasma membranes and non-labeled reindeer pancreas-derived insulin.

### Preparing radiolabeled hormone

The radioreceptor insulin test-system makes use of plasma membranes of rat liver isolated according to Neville's technique (providing a 15-20 times enrichment of the membranes with receptors).

The reindeer pancreas-derived insulin (the test preparation) is purified as follows. Successive procedures of extraction, ion-exchange chromatography and isoelectric precipitation enable one to obtain coarse insulin fractions which upon lyophilization are fractionated with the aid of high-efficiency reversed-phase liquid co-chromatography. A system of buffers makes it possible to conduct a single-stage fractionation of the pancreas-derived insulin.

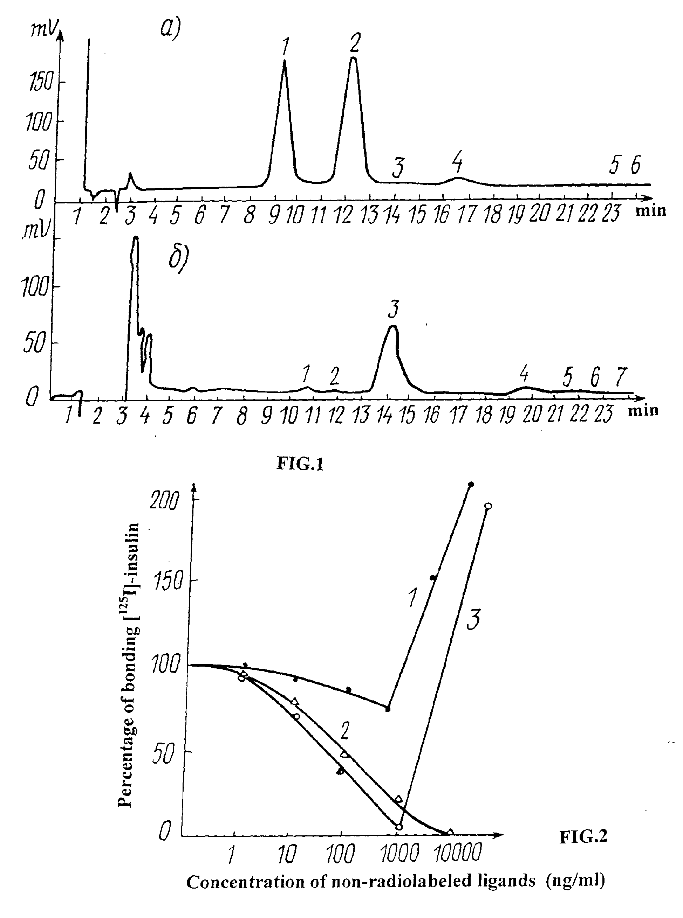

"Reference" chromatograms of a mixture of commercial bovine insulin (peak #.1) and porcine insulin (peak #2) are taken under conditions similar to those the test preparation, the elution profiles for which being presented in FIG.1A.

The elution profiles for a reindeer pancreas-derived insulin is presented in FIG.1B (peak #3).

A chromatogram of the test preparation makes possible analyzing its physical-chemical properties. As is evident from FIG.1 (A, B) the resolution of the high-efficiency reversed-phase liquid co-chromatography system used makes it possible to separate insulin whose structure differs in two amino acids, i.e., high homogenicity of the resultant peptides can be spoken of.

Amount of "mobility" (Rf) of significant fractions of the test preparation calculated as per standard bovine insulin and porcine insulin are tabulated below.

**Table Mobility of separate fractions of test specimens**

| | Reindeer insulin | |
|---|---|---|
| | Column hold-up time, min | Rf |
| Bovine insulin | 9.28 | 1 |
| Porcine | 12.20 | 0.76 |

| | | |
|---|---|---|
| insulin | | |
| Peak #1 | 10.81 | 0.86 |
| Peak #2 | - | - |
| Peak #3 | 14.23 | 0.65 |
| Peak #4 | 19.73 | 0.47 |

As is evident from the table above, column hold-up time comparison lends support to an absence of any similarity of the reindeer pancreas-derived insulin to both the standard insulin derived from large horned cattle (i.e., bovine insulin) and the porcine insulin. Reindeer insulin (in its major fraction) features hydrophobicity higher than that of standard insulins and hence definite differences in the structure a molecule thereof.

A coarse specimen of reindeer pancreas-derived insulin is assessed by means of *in vitro* experiments against its ability to cause a hypoglycemic effect.

Experimental groups of test young rats are given intraperitoneal injections of reindeer insulin (in a dose of 50 or 100 µg) dissolved in 1.5 ml of physiological saline. 20 min after insulin administration in a dose of 50 µg there is noted dystaxia in the test animals, and with a dose of 100 µg intense convulsions are observed which are stopped by glucose administration. Convulsions in test rats arising in response to an injection of reindeer Zn-insulin preparation during *an in vivo* experiment followed by ceasing the effect of the preparation by intraperitoneal glucose administration give an unambiguous evidence of a hypoglycemic effect produced by said preparation. Such a technique is a standard express-test applicable for assessing biological potency of any insulin.

Interactability of separate fractions of reindeer insulin with the insulin receptor of the rat lever plasma membranes is illustrated in FIG.2.

As can be seen from FIG.2, two fractions of reindeer insulin (peak #1-2 and peak #3) are capable of displacing, in a dose-dependent mode, the receptor-bonded radiolabeled hormone. Moreover, it is in the range of concentrations of the peak #3 peptide from 0,1 to 100 ng/ml that the run of displacement curves of standard insulin and of the fraction #3 under test is virtually the same, whereas with a peptide concentration above 100 ng/ml an abrupt increase in bonding the radiolabeled hormone is observed.

The fact gives evidence that a factor is present in all tested peptide fractions which promotes bonding the hormone with the insulin receptor.

None of the heretofore-known kinds of insulin of vertebrates has such an activating ability.

### Industrial Applicability

The present invention can find application in the medical industry for insulin production.

## Claims

1. A method for producing insulin **CHARACTERIZED in that** a reindeer pancreas is homogenized in an ethanol hydrochloride solution (1:15), extracted for a two-hour period at room temperature and centrifugation-clarified, a supernatant fluid is subjected to ion-exchange chromatography involving the use of KY-23 microporous sulfocationite in the H⁺ form, an isoelectric precipitation of the proteins concomitant with the principal matter is performed by successive reduction of the pH value of the eluate resultant from ion-exchange chromatography to 2.0, and further upon adding acetone (20% of a total amount of the solution), to 4.0, whereupon the mixtures are let to stand for an hour at +20°C and then to stand overnight at +4°C, next a 10-percent zinc- acetate solution (2% of a total amount of the solution) is added to the supernatant fluid to bring the pH value of the solution to 5.95, after which the solution is let to stand at +20°C for an hour and then put in a cooler for 72 hours, the precipitate is centrifugation-separated to produce insulin which is then purified by high-efficiency reversed-phase liquid co-chromatography.

2. Insulin **CHARACTERIZED in that** it is produced as disclosed in claim 1 and is capable of competing for a bond with an insulin receptor when in a concentration over 100 ng/ml.
